# EUROPEAN PATENT SPECIFICATION

(11) **EP 3 785 764 B1**
(45) Date of publication and mention of the grant of the patent: **21.08.2024**
(21) Application number: 19794072.9
(22) Date of filing: 19.04.2019
(51) Int. Cl.: A61N 2/02, A61N 2/00

(54) **SYSTEM FOR PHYSIOTHERAPEUTIC CORRECTION OF HUMAN SLEEP**
SYSTEM ZUR PHYSIOTHERAPEUTISCHEN KORREKTUR DES MENSCHLICHEN SCHLAFES
SYSTÈME DE CORRECTION PHYSIOTHÉRAPEUTIQUE ET DE THÉRAPIE DU SOMMEIL D'UNE PERSONNE

(30) Priority: 24.04.2018 RU 2018115258
(43) Date of publication of application: 03.03.2021
(73) Proprietor: Obschestvo s Ogranichennoi Otvetstvennostyu "Tsentr Neirotekhnology Sna I Bodrstvovania" (Ooo "TSNSIB"), Moscow, 121205 (RU)
(72) Inventor: BLOKHIN, Ilya Sergeevich, Zelenograd, 124617 (RU); DOROKHOV, Vladimir Borisovich, Mytischi, 141001 (RU); BLOKHIN, Alexey Sergeevich, Zelenograd, 124460 (RU)
(74) Representative: Chimini, Francesco
(86) International application number: PCT/RU2019/000263
(87) International publication number: WO 2019/209142

(56) References cited:
- CN-A- 1 116 555
- CN-A- 103 893 915
- RU-C1- 2 125 473
- RU-C1- 2 125 473
- RU-C2- 2 496 533
- RU-C2- 2 649 519
- RU-U1- 120 878
- US-A1- 2010 298 624
- US-A1- 2014 046 116
- US-A1- 2014 046 116
- US-A1- 2014 303 425
- US-A1- 2017 028 166
- US-A1- 2018 056 083

## Description

### Field of the invention

This invention relates to a system of physiotherapeutic correction and therapy of human sleep.

### Background of the invention

There are various devices for normalizing human sleep.

Thus, Russian patent No. 2170109 (publ. 10.07.2001) describes a device for elimination of sleep disorders, wherein a pulsed low-frequency quasistationary electrostatic field is used. This device requires the placement of several electrodes on the human body, which is very inconvenient, since the sleeper often turns.

U.S. patent No. 8517909 (publ. on 27.08.2013) describes devices and methods for improving sleep, reducing pain and encouraging naturaltreatment. In this solution, a plurality of simultaneous ultra-low-frequency mechanical vibration waves is formed for a patient lying on a table or sitting in an arm-chair. This method can only be used in a hospital or clinic.

Russian patent application No. 94003463 (publ. on 27.07.1996) describes a device for physiotherapeutic influence, intended for rendering the physiotherapeutic assistance in case of pain effects (i.e. for alleviation of pain). The impact is carried out by a rotating permanent magnet, which does not enable it to be used for sleep therapy.

U.S. patent No. 5441528 (publ. on 15.08.1995) discloses a method and system for applying low-energy emission therapy by means of a high-frequency electromagnetic field with a frequency of 1 GHz, modulated by a low frequency in the range of 0.1 Hz - 10 kHz.

The closest analogue of this invention is U.S. patent No. 8808159 (publ. on 19.08.2014). It describes a device for stimulating local and higher-level autoreg-ulatory mechanisms in the body, it effects by magnetic field pulses with a frequency of 8-15 Hz and an intensity of 30-150 µT. Such magnetic field intensities exceed the hygienic safety standards of the Russian Federation. In addition, this document does not comprise a detailed description of the patented device itself.

Document US2018/056083A1 discloses Repetitive Transcranial Magnetic Stimulation (rTMS) is administered at a pulse frequency that is optimized with respect to settling time of the EEG oscillations, measured at the pulse frequency.

### Summary of the invention

This invention solves the problem of overcoming the disadvantages of the closest analogue and ensures the achievement of a technical result in the form of an expanding the range of technical means.

The present invention provides a system according to claim 1. Further aspects and preferred embodiments are defined in the dependent claims.

In the object of this invention, a system for physiotherapeutic correction and therapy of human sleep is claimed, comprising: a pulse generator adapted to generate pulses with a configurable repetition frequency in the range from 1 to 40 Hz; a magnetic field former connected to the pulse generator; a feedback means designed to adjust the repetition frequency of the pulses generated.

A feature of the system according to the object of this invention is that the pulse generator can be configured to generate rectangular pulses.

Another feature of the system according to the object of this invention is that the magnetic field former can be configured to create a magnetic field with an intensity of up to 20 µT.

Another feature of the system according to the object of this invention is that the magnetic field former can be based on three inductor coils placed in three orthogonal planes, one of which is parallel to the person's bed plane.

Another feature of the system according to the object of this invention is that the feedback means can be performed based on a smartphone with a software application designed to set the desired repetition frequency correction mode of the generated pulses during the forthcoming person's sleep.

Finally, another feature of the system according to the object of this invention consists in the fact that the said feedback means can be implemented based on a smartphone with a software application for correcting the repetition frequency of the generated pulses based on data from at least one of the following means: a heart rate monitor, an accelerometer, a respiration rate meter, wherein the means is placed on or near the person.

### Brief description of the drawings

This invention is illustrated in the figures attached.
Fig. 1 shows a scheme of the system according to the invention.
Fig. 2 shows the signals generated by the system according to the Fig. 1.

### Detailed description of embodiments of the invention

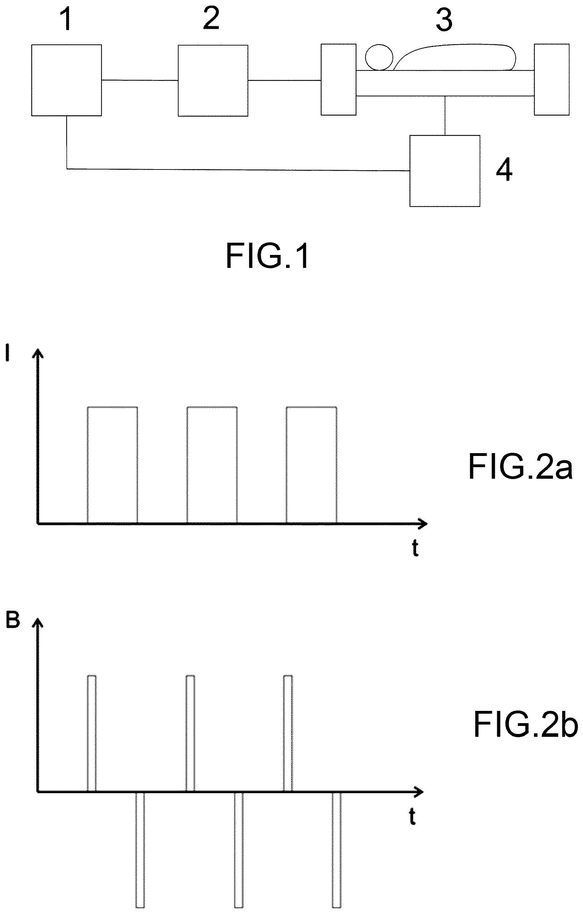

An examplary method can be implemented using the system according to the object of this invention, the scheme of which is presented in Fig. 1.

The system of physiotherapeutic correction and therapy of sleep comprises a pulse generator 1 configured to generate pulses with a configurable repetition frequency in the range from 1 to 40 Hz, and a magnetic field generator 2 connected to the pulse generator 1. Any suitable device can be used as a pulse generator 1, which is known to those skilled in the art. Preferably, the pulse generator 1 generates rectangular pulses (Fig. 2a).

The magnetic field former 2 can be made in the form of an inductor coil placed in a plane, which is parallel to the plane of the bed 3 of the person who needs in a physiotherapeutic correction or therapy of sleep. However, the magnetic field former 2 is preferably made on the basis of three inductor coils placed in three orthogonal planes, one of which is parallel to the plane of the person's bed 3. For example, these three inductor coils can be placed on three adjacent sides of a cube.

The former 3 of the magnetic field is configured to create a magnetic field with an intensity of up to 20 µT, which does not exceed the permissible hygienic safety standards of the Russian Federation. Wherein, in the zone of the bed 3, where a person is located, the intensity of the magnetic field does not exceed 30 nT. It should be noted, that when the pulse generator 1 is implemented in the above-mentioned preferred embodiment, the magnetic field B occurs only at those moments that correspond to the edges of the current pulses (Fig. 2b)

In addition to these components, the system of the invention includes the feedback means 4 for adjusting the pulse repetition frequency from the pulse generator 1. The implementation of the feedback means 4 may be different.

For example, this feedback means 4 can be based on a smartphone with a software application designed to set the required repetition frequency correction mode of the generated pulses during the forthcoming sleep of the person. In this case, feedback can be carried out through a survey. The corresponding software application can be installed on a smartphone or tablet on iOS and Android platforms. The survey will take place in the format of the modified standard questionnaires for the quality of sleep and dreams. That is, after waking up, the user will be asked to answer several control questions. By answering these questions, the application will "understand" the extent of effectiveness of the impact of the system of this invention. And with the accumulation of statistics, this software application will "know" the need of this particular user.

In more advanced versions of the device, a more "rigid" sleep quality control tool will be possible to use. This can be achieved by implementing the feedback means 4 based on a smartphone with a software application for correcting the repetition frequency of the generated pulses based on data from at least one of the following means: a heart rate monitor, an accelerometer, a respiration rate meter. This means can be placed on a person (for example, on his hand) or near him.

The operation of the system according to the object of this invention for implementing the exemplary method is obvious. A magnetic field former 2 is installed near the bed 3 of a person who needs correction or therapy of sleep. The pulse generator 1 generates pulses with a configurable repetition frequency in the range from 1 to 40 Hz. These pulses are fed to the magnetic field former 2 placed at a distance of 50 to 200 cm from the person. The frequency of the pulses generated is adjusted based on the feedback from the feedback means 4.

This invention is based on non-contact remote exposure to weak electromagnetic fields of ultra-low frequency (EMF ULF) which penetrate freely the body tissues. These fields hold a special place among natural environmental factors, which is associated with the interaction of the Earth's atmosphere and the Sun. Unlike other, more high-frequency electromagnetic fields, a noteworthy feature of the EMF ULF range is its coincidence with the frequency characteristics of the bioelectric potentials of organs and tissues of humans and animals, which provides the possibility of "resonant" interaction of EMF ULF with living organisms.

The feedback is implemented using the so-called sleep tracker, which is, for example, a pulse meter + motion sensor. In other cases, a noise analyzer, an eye movement sensor, a brain electroencephalogram recorder can be used. Various sleep trackers are heavily marketed and their specific implementation is not included in the scope of claims of this invention.

Data from the sleep tracker is fed to a mobile application that controls the operation modes of the pulse generator 1 in accordance with the measured parameters of the sleeper. This mobile application indicates the dynamics of the phases of sleep, the dynamics of exposure, as well as local and global statistics. It can accommodate the functions of a "smart alarm clock", a target scheduler (for example, for developing sleep modes). The mobile application can synchronize data on sleep quality with a remote server, where the user can access the WEB interface and the personal sleep manager, which is the user's personal account in the WEB interface system, which presents his sleep quality data. Based on statistics and goals, the personal manager can create personalized exposure modes for the pulse generator 1 (for example, if the user wants to sleep less and deeper, or, conversely, to see many bright dreams and wake up easily in the morning; if the user wants to change the sleep mode, time of falling asleep and awakening, etc.). A separate function of a personal manager is communication with a personal expert (in accordance with the subscription fees on the WEB interface), who will have access to personal user statistics and solve more complex personal tasks for normalizing sleep.

It should be noted separately that due to the small size of the pulse generator 1 and the magnetic field former 2, the system of this invention is transportable and can be used with an independent power source (for example, in an airplane).

Thus, this invention expands the range of technical means by creating a simple and easy-to-use system that enables to increase the range of uses.

## Claims

1. A system of physiotherapeutic correction and therapy for human sleep of a person, comprising:
- a pulse generator (1) adapted to generate pulses with a configurable repetition frequency in the range from 1 to 40 Hz;
- a magnetic field former (2) connected to said pulse generator,
- a feedback means (4) designed to adjust said repetition frequency of the generated pulses, **characterized in that**
said magnetic field former is configured to create a magnetic field with an intensity of up to 20 µT,
said magnetic field former is implemented on the basis of three inductor coils placed in three orthogonal planes, one of which is parallel to the plane of the person's bed (3), and
said feedback means are sleep trackers.

2. The system according to claim 1, wherein said pulse generator is configured to generate rectangular pulses.

3. The system according to claim 1, wherein said feedback means are based on a smartphone with a software application designed to set a required repetition frequency correction mode of the generated pulses during a forthcoming sleep of said person.

4. The system according to claim 1, wherein said feedback means are based on a smartphone with a software application for correcting the repetition frequency of the generated pulses based on data from at least one of the following means: a heart rate monitor, an accelerometer, a respiration rate meter, wherein the means is adapted to be placed on or near said person.

## Patentansprüche

1. System zur physiotherapeutischen Korrektur und Therapie für menschlichen Schlaf einer Person, umfassend:
- einen Impulsgenerator (1), der angepasst ist, Impulse mit einer konfigurierbaren Wiederholungsfrequenz in dem Bereich von 1 bis 40 Hz zu generieren;
- einen Magnetfeldformer (2), der mit dem Impulsgenerator verbunden ist,
- ein Feedback- bzw. Rückkopplungsmittel (4), das ausgelegt ist, die Wiederholungsfrequenz der generierten Impulse anzupassen bzw. einzustellen, **dadurch gekennzeichnet, dass**
der Magnetfeldformer konfiguriert ist, ein Magnetfeld mit einer Intensität von bis zu 20 µT zu erzeugen,
der Magnetfeldformer auf der Basis von drei Induktorspulen implementiert ist, die in drei orthogonalen Ebenen platziert sind, von denen eine parallel zu der Ebene des Bettes (3) der Person ist, und
die Feedback-Mittel Schlaf-Tracker sind.

2. System nach Anspruch 1, wobei der Impulsgenerator konfiguriert ist, Rechteckimpulse zu generieren.

3. System nach Anspruch 1, wobei die Feedback-Mittel auf einem Smartphone mit einer Softwareanwendung basieren, die ausgelegt ist, einen erforderlichen Wiederholungsfrequenzkorrekturmodus der generierten Impulse während eines bevorstehenden Schlafs der Person festzulegen.

4. System nach Anspruch 1, wobei die Feedback-Mittel auf einem Smartphone mit einer Softwareanwendung zum Korrigieren der Wiederholungsfrequenz der generierten Impulse basierend auf Daten von zumindest einem der folgenden Mittel basieren: einem Herzfrequenzmonitor, einem Beschleunigungsmesser bzw. -sensor, einem Atemfrequenzmesser bzw. -sensor, wobei das Mittel angepasst ist, an oder in der Nähe der Person platziert zu werden.

## Revendications

1. Système de correction physiothérapeutique et de thérapie du sommeil humain d'une personne, comprenant :
- un générateur d'impulsions (1), adapté pour générer des impulsions selon une fréquence de répétition configurable dans la plage de 1 à 40 Hz ;
- un dispositif de formation de champ magnétique (2) connecté audit générateur d'impulsions,
- un moyen de rétroaction (4) conçu pour ajuster ladite fréquence de répétition des impulsions générées, **caractérisé en ce que**
ledit dispositif de formation de champ magnétique est configuré pour créer un champ magnétique présentant une intensité pouvant atteindre les 20 µT,
ledit dispositif de formation de champ magnétique est mis en oeuvre sur la base de trois bobines d'induction placées dans trois plans orthogonaux, l'un d'eux étant parallèle au plan du lit (3) de la personne, et
ledit moyen de rétroaction étant un capteur de sommeil.

2. Système selon la revendication 1, dans lequel ledit générateur d'impulsions est configuré pour générer des impulsions rectangulaires.

3. Système selon la revendication 1, dans lequel ledit moyen de rétroaction repose sur un smartphone comportant une application logicielle conçue pour définir un mode de correction de fréquence de répétition requis des impulsions générées pendant un sommeil à venir de ladite personne.

4. Système selon la revendication 1, dans lequel ledit moyen de rétroaction est basé sur un smartphone comportant une application logicielle qui permet de corriger la fréquence de répétition des impulsions générées sur la base de données provenant d'au moins l'un des moyens suivants : un moniteur de fréquence cardiaque, un accéléromètre, un compteur de fréquence respiratoire, le moyen étant adapté pour être placé sur, ou près, de ladite personne.
